(19) Europäisches Patentamt
European Patent Office
Office européen des brevets

(11) EP 3 011 959 A1

(12) **EUROPEAN PATENT APPLICATION**

(43) Date of publication:
**27.04.2016 Bulletin 2016/17**

(51) Int Cl.:
***A61K 31/14*** *(2006.01)* ***A61P 29/00*** *(2006.01)*
***A61P 27/00*** *(2006.01)* ***A61P 27/02*** *(2006.01)*

(21) Application number: **14190172.8**

(22) Date of filing: **23.10.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Santen SAS**
**91000 Evry (FR)**

(72) Inventor: **Daull, Philippe**
**91450 Soisy-sur-Seine (FR)**

(74) Representative: **Icosa**
**83 avenue Denfert-Rochereau**
**75014 Paris (FR)**

(54) **Quaternary ammonium compound for use as an inhibitor of protein kinase C alpha**

(57) This invention relates to quaternary ammonium compound for use as an inhibitor of protein kinase C alpha, of formula I:

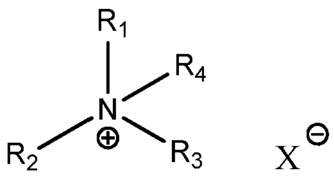

(I)

wherein:
$R_1$ is selected from the group of alkyl, alkene, aryl or alkylaryl group; preferably $R_1$ is a benzyl, alkylbenzyl or alkylphenyl group; more preferably benzyl or methylphenyl group; optionally substituted by hydroxyl, oxo, nitro, amido, carboxy, amino, cyano, alkoxy, alkyl, alkene, alkyne, ester or heteroalkyl group.
$R_2$ is an alkyl chain comprising at least 12 carbon atoms; preferably comprising 14, 16 or 18 carbon atoms;
$R_3$ or $R_4$ are identical or different and each is selected from an alkyl chain; preferably a C1-C4 alkyl chain; more preferably is methyl group.
$X^-$ is selected from an anion of a pharmaceutically acceptable acid or from halo group; preferably $X^-$ is selected from halo group; more preferably is $Cl^-$ or $Br^-$. In particular, the present invention relates to a long-chain quaternary ammonium of formula (I) as a wound healing and/or an anti-inflammatory agent for use in the treatment of eye conditions.

EP 3 011 959 A1

## Description

### FIELD OF INVENTION

The present invention relates to the use of long-chain quaternary ammonium as an inhibitor of protein kinase C alpha. Especially, the present invention relates to a long-chain quaternary ammonium which is an inhibitor of protein kinase C alpha for use in wound healing and/or in the treatment of inflammatory conditions. More particularly, this invention relates to composition containing such quaternary ammonium for the treatment of eye conditions.

### BACKGROUND OF INVENTION

In the ophthalmic area, inflammation turns out to be a real plague causing most of the time an alteration of iris, ciliary body, cornea, or conjunctiva. Especially, in the recent past, experimental and clinical studies have shown that the long-term use of drugs for the treatment of ocular diseases or conditions may induce major and frequent ocular surface disorders and inflammatory reactions.

Whereas toxicity is defined as the degree to which a substance can harm humans or animals, inflammation encompasses all the phenomena arising after an aggression caused by an injury, an infection or a trauma following a surgical treatment. Inflamed tissues are usually characterized by redness, heat, swelling and/or pain.

In general, the ophthalmic medicines are solutions administered under eye-drops or intraocular forms comprising most frequently a quaternary ammonium, such as benzalkonium chloride, playing the role of preservative agent. This allows formulation to fulfill the preservation requirements along their shelf life and upon use.

For example, benzalkonium chloride is a nitrogenous cationic surface-acting agent belonging to the quaternary ammonium group. Benzalkonium chloride is defined as a mixture of compounds of general formula $C_6H_5CH_2N(CH_3)_2RCl$, wherein R is an alkyl group mostly having 12, 14 and 16 carbons.

However some recent studies have highlighted the involvement of quaternary ammonium compounds in cell toxicity and inflammation disorders for preserved ophthalmic formulations (Baudouin, C. et al, Preservatives in eye drops: the good, the bad and the ugly. Prog. Retin. Eye Res., July 2010, Vol.29, N°4, pp 312-334; Debbasch, C.et al., Quaternary ammoniums and other preservatives contributions in oxidative stress and apoptosis on Chang conjunctival cells, Inv. Ophtalmo. Vis. Sci., March 2001, Vol. 42, N°3, pp 642-652 and Vlachy, N. et al., Detremining the cytotoxicity of cationic surfactant mixtures on HeLa cells, Colloids Surf. B Biointerfaces, May 2009, Vol.70, N°2, pp 278-280).

Indeed, the use of high concentrations and repeated administration of quaternary ammonium favor the development of iatrogenic side effects with evidences of toxicity reactions, such as delayed wound healing, through the elevation of pro-inflammatory and/or pro-apoptotic mediators, oxidative stress or disruption of cellular junctions, ultimately leading to discomfort for the patients.

Therefore, there is a need to provide: 1) ophthalmic compositions that will not result in the development of iatrogenic side effects upon their instillation, by opposition to the benzalkonium chloride preserved eye drops; and 2) ophthalmic compositions that will promote the corneal epithelial wound healing process as a consequence of their ability to manage the ocular inflammation reactions caused by quaternary ammonium-preserved eye drops. Furthermore, there is a need to provide means to combat the iatrogenic side effects induced by the eye drops-administered compounds other than quaternary ammonium.

Filion et al (1997) reported that cationic lipids containing a quaternary ammonium moiety were able to reduce inflammatory effects through the inhibition of protein kinase C ("Anti-inflammatory activity of cationic lipids", British Journal of Pharmacology, 122, 1997, pp 551-557). However, the anti-inflammatory activity of those cationic lipids was evaluated following intraperitoneal or footpad injections. Filion et al. have not studied the activity of quaternary ammonium for the manufacturing of ophthalmic compositions.

Other examples also reported an anti-inflammatory effect when using solutions of quaternary ammonium for specific treatments of skin disorders.

EP 1 620 063 discloses composition comprising vanilla extract and a mixture of a cationic chloride salt and a quaternary ammonium for the treatment of skin disorders. In EP 1 620 063, the active agent for anti-allergic and anti-inflammatory therapy is the vanilla extract. It was reported that the association with the quaternary ammonium improved the anti-inflammatory effects of the extract. However the exact mechanism of action of quaternary ammonium and its association with vanilla extract is not known and described by the authors. In that particular case, quaternary ammonium may as well only have a carrier effect, as it helps the active agent's penetration in the skin, de facto increasing the delivered dose and hence the efficacy of the active agent.

US2010/0278906 refers to an antimicrobial solution for use on the skin, comprising quaternary ammonium chloride. In US2010/0278906, quaternary ammonium compounds are used as biocidal agents, which is the classical use for such compounds. In this case the reduction of inflammation is the consequence of the anti-infection activity (bactericidal) of the quaternary ammoniums. In US2010/0278906, decreasing of inflammation is also achieved by the

absence or the reduction of potentially irritating excipients: polysorbates, ethanol or anionic compounds, in the final composition. In particular, preferred compositions of US2010/0278906 comprise water soluble and non-benzylcationic compounds. Thus, US2010/0278906 does not disclose the use of quaternary ammonium, preferably quaternary benzylammonium halide, as anti-inflammatory agents for ophthalmic compositions by a mechanism of action linked to PKC activity.

Quaternary ammonium containing-compositions (emulsions) were also used in the ophthalmic field, in particular to increase the residence time of the cationic emulsions onto the ocular surface, as an improved drug delivery vehicle (Daull et al., Cornea 2013, 32, pp 345-354). The cationic emulsions were very well tolerated by the ocular surface (Liang et al., Molecular Vision 2008, 14, pp 204-214), and no evidence of anti-inflammatory properties of the quaternary ammonium via a mechanism of action linked to PKC activity was discussed and established.

Corneal epithelial wound healing is a dynamic process regulated through distinct signaling pathways lead by tyrosine kinase receptors, and serine threonine kinases including PKCs. Previous studies have demonstrated the involvement of PKCs in cell adhesion and wound healing processes (Lin and Bazan, 1995; Breitkreutz et al, 2007) and some pharmaceutical compositions have been developed for accelerating the wound healing process thanks to a PKCs inhibitor (WO 2012/063237; EP 2 526 953) but none of these studies disclosed the use of quaternary ammonium as inhibitor of PKCs.

Surprisingly, the Applicant demonstrated that at very low concentration, some particular quaternary ammonium inhibit the protein kinase C alpha (PKC-$\alpha$) and thus, act as anti-inflammatory and/or a wound healing agents.

## SUMMARY

This invention thus relates to quaternary ammonium compound for use as an inhibitor of protein kinase C alpha, of formula I:

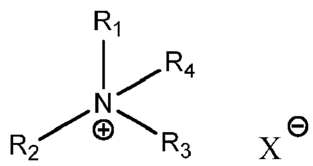

(I)

wherein:

$R_1$ is selected from the group of alkyl, alkene, aryl or alkylaryl group; preferably $R_1$ is a benzyl, alkylbenzyl or alkylphenyl group; more preferably benzyl or

methylphenyl group; optionally substituted by hydroxyl, oxo, nitro, amido, carboxy, amino, cyano, alkoxy, alkyl, alkene, alkyne, ester or heteroalkyl group;

$R_2$ is an alkyl chain comprising at least 12 carbon atoms; preferably comprising 14, 16 or 18 carbon atoms;

$R_3$ or $R_4$ may be identical or different and each is selected from an alkyl chain; preferably C1-C4 alkyl chain; more preferably methyl group;

X- is selected from an anion of a pharmaceutically acceptable acid or from halo group; preferably X- is selected from halo group; more preferably is $Cl^-$ or $Br^-$.

According to one embodiment, quaternary ammonium is not dioctadecyldimethylammonium bromide.

In particular, the invention relates to a quaternary ammonium compound for use as an inhibitor of protein kinase C alpha of formula I selected from the group of quaternary ammonium halide; preferably cetalkonium chloride (CKC).

According to a first embodiment, the quaternary ammonium compound is for use in a wound healing and/or in treating inflammatory conditions.

The invention also relates to composition comprising at least one quaternary ammonium compound for use as an inhibitor of protein kinase C alpha of formula (I). Especially, the invention refers to composition wherein inhibitor of protein kinase C alpha is cetalkonium chloride (CKC).

According to one embodiment, said composition is for use in the treatment of eye conditions; especially, in

ocular surface diseases including corneal and conjunctival lesions.

According to one embodiment, said composition comprises quaternary ammonium compound for use as an inhibitor of protein kinase C alpha of formula (I) in association with an ophthalmically acceptable excipient.

The present invention also relates to a pharmaceutical composition or a medicament comprising the quaternary ammonium compound for use as inhibitor of protein kinase C alpha of formula (I).

According to one embodiment, said composition, pharmaceutical composition or medicament is topically administered through any form selected from solution, dispersion, emulsion, eye drops, artificial tears, gel, ointment, patch, film or suspension suitable for ophthalmic use.

According to one embodiment, said composition, pharmaceutical composition or medicament is packaged in unitary doses or in multidose containers.

This invention also relates, in a first aspect, to a method of treating inflammation on tissue and/or an organ comprising administering to the tissue and/or the organ of a subject in need thereof a composition comprising a therapeutically effective amount of an inhibitor of protein kinase C alpha according to formula (I).

This invention also relates, in a second aspect, to a method of treating a wound comprising administering to a subject in need thereof a composition comprising a therapeutically effective amount of an inhibitor of protein kinase C alpha according to formula (I).

This invention also relates, in a third aspect, to a method of inhibiting expression of IL17 from CD4+ T-helper cells, comprising contacting said cells with a composition comprising a therapeutically effective amount of an inhibitor of protein kinase C alpha according to formula I:

In one embodiment, the therapeutically effective amount is greater than 0% to 0.3% of the composition. In one embodiment, the therapeutically effective amount is 0.002 % to 0.3% of the composition. In one embodiment, the therapeutically effective amount is greater than 0% to less than 0.002% of the composition. In the method of the invention, the composition may be topically administered to the tissue and/or organ in a form selected from group consisting of a powder, a liposomal form, an ointment, a gel and a patch. In the method of the invention, the organ may be an eye. In the method of the invention, the composition may be in a form selected from the group consisting of a solution, a dispersion, an emulsion, eye drops, artificial tears, an ointment, a gel, a patch, a film suitable for ophthalmic use and a suspension suitable for ophthalmic use. In the method of the invention, the composition may be administered by injection to the tissue and/or organ in a form selected from group consisting of a sterile aqueous solution, a dispersion, an emulsion and a suspension. In an embodiment, the inhibitor of protein kinase C alpha according to formula I is the only inhibitor of protein kinase C alpha according to formula I administered. In one embodiment, $R_2$ of the inhibitor of protein kinase C alpha according to formula I is 14, 16 or 18 atoms. In one embodiment, $R_2$ is 16. In one embodiment, $R_3$ and $R_4$ of the inhibitor of protein kinase C alpha according to formula I are identical or different and each is selected from C1 to C4 alkyls. In one embodiment, X- is a halo group selected from the group consisting of Cl-and Br-.

## DETAILED DESCRIPTION

As mentioned above, this invention relates to a quaternary ammonium compound for use as an inhibitor of PKC alpha of formula I:

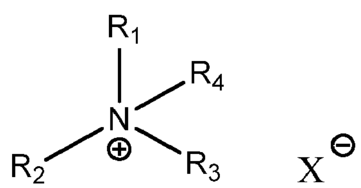

(I)

wherein $R_1$, $R_2$, $R_3$ and $R_4$ are as defined above.

Especially, the invention relates to an inhibitor of PKC alpha of formula (I) for use in wound healing and/or in treating inflammatory conditions.

According to one embodiment, the present invention comprises compounds selected from preferred compounds 1 to 51.

| Cpd n° | R1 | R2 | R3 | R4 | X |
|---|---|---|---|---|---|
| 1 | $-CH_2-C_6H_5$ | $-(CH_2)_{15}-CH_3$ | $-CH_3$ | $-CH_3$ | Cl |
| 2 | $-CH_2-C_6H_5$ | $-(CH_2)_{15}-CH_3$ | $-CH_3$ | $-CH_2CH_3$ | Cl |
| 3 | $-CH_2-C_6H_5$ | $-(CH_2)_{15}-CH_3$ | $-CH_2CH_3$ | $-CH_3$ | Cl |
| 4 | $-CH_2-C_6H_5$ | $-(CH_2)_{15}-CH_3$ | $-CH_3$ | $-(CH_2)_2CH_3$ | Cl |
| 5 | $-CH_2-C_6H_5$ | $-(CH_2)_{15}-CH_3$ | $-(CH_2)_2CH_3$ | $-CH_3$ | Cl |
| 6 | $-CH_2-C_6H_5$ | $-(CH_2)_{15}-CH_3$ | $-CH_3$ | $-CH(CH_3)_2$ | Cl |
| 7 | $-CH_2-C_6H_5$ | $-(CH_2)_{15}-CH_3$ | $-CH(CH_3)_2$ | $-CH_3$ | Cl |
| 8 | $-CH_2-C_6H_5$ | $-(CH_2)_{15}-CH_3$ | $-CH_3$ | $-(CH_2)_3CH_3$ | Cl |
| 9 | $-CH_2-C_6H_5$ | $-(CH_2)_{15}-CH_3$ | $-(CH_2)_3CH_3$ | $-CH_3$ | Cl |
| 10 | $-CH_2-C_6H_5$ | $-(CH_2)_{15}-CH_3$ | $-CH_3$ | *iso*-Butyl | Cl |
| 11 | $-CH_2-C_6H_5$ | $-(CH_2)_{15}-CH_3$ | *iso*-Butyl | $-CH_3$ | Cl |
| 12 | $-CH_2-C_6H_5$ | $-(CH_2)_{15}-CH_3$ | $-CH_3$ | *tert*-Butyl | Cl |
| 13 | $-CH_2-C_6H_5$ | $-(CH_2)_{15}-CH_3$ | *tert*-Butyl | $-CH_3$ | Cl |
| 14 | $-(CH_2)_2-C_6H_5$ | $-(CH_2)_{15}-CH_3$ | $-CH_3$ | $-CH_3$ | Cl |
| 15 | $-(CH_2)_2-C_6H_5$ | $-(CH_2)_{15}-CH_3$ | $-CH_3$ | $-CH_2CH_3$ | Cl |
| 16 | $-(CH_2)_2-C_6H_5$ | $-(CH_2)_{15}-CH_3$ | $-CH_2CH_3$ | $-CH_3$ | Cl |
| 17 | $-(CH_2)_2-C_6H_5$ | $-(CH_2)_{15}-CH_3$ | $-CH_3$ | $-(CH_2)_2CH_3$ | Cl |
| 18 | $-(CH_2)_2-C_6H_5$ | $-(CH_2)_{15}-CH_3$ | $-(CH_2)_2CH_3$ | $-CH_3$ | Cl |
| 19 | $-(CH_2)_2-C_6H_5$ | $-(CH_2)_{15}-CH_3$ | $-CH_3$ | $-CH(CH_3)_2$ | Cl |
| 20 | $-(CH_2)_2-C_6H_5$ | $-(CH_2)_{15}-CH_3$ | $-CH(CH_3)_2$ | $-CH_3$ | Cl |
| 21 | $-(CH_2)_2-C_6H_5$ | $-(CH_2)_{15}-CH_3$ | $-CH_3$ | $-(CH_2)_3CH_3$ | Cl |
| 22 | $-(CH_2)_2-C_6H_5$ | $-(CH_2)_{15}-CH_3$ | $-(CH_2)_3CH_3$ | $-CH_3$ | Cl |
| 23 | $-(CH_2)_2-C_6H_5$ | $-(CH_2)_{15}-CH_3$ | $-CH_3$ | *iso*-Butyl | Cl |
| 24 | $-(CH_2)_2-C_6H_5$ | $-(CH_2)_{15}-CH_3$ | *iso*-Butyl | $-CH_3$ | Cl |
| 25 | $-(CH_2)_2-C_6H_5$ | $-(CH_2)_{15}-CH_3$ | $-CH_3$ | *tert*-Butyl | Cl |
| 26 | $-(CH_2)_2-C_6H_5$ | $-(CH_2)_{15}-CH_3$ | *tert*-Butyl | $-CH_3$ | Cl |
| 27 | $-CH_2-C_6H_5$ | $-(CH_2)_{17}-CH_3$ | $-CH_3$ | $-CH_3$ | Cl |
| 28 | $-CH_2-C_6H_5$ | $-(CH_2)_{17}-CH_3$ | $-CH_3$ | $-CH_2CH_3$ | Cl |
| 29 | $-CH_2-C_6H_5$ | $-(CH_2)_{17}-CH_3$ | $-CH_2CH_3$ | $-CH_3$ | Cl |
| 30 | $-CH_2-C_6H_5$ | $-(CH_2)_{17}-CH_3$ | $-CH_3$ | $-(CH_2)_2CH_3$ | Cl |
| 31 | $-CH_2-C_6H_5$ | $-(CH_2)_{17}-CH_3$ | $-(CH_2)_2CH_3$ | $-CH_3$ | Cl |
| 32 | $-CH_2-C_6H_5$ | $-(CH_2)_{17}-CH_3$ | $-CH_3$ | $-CH(CH_3)_2$ | Cl |
| 33 | $-CH_2-C_6H_5$ | $-(CH_2)_{17}-CH_3$ | $-CH(CH_3)_2$ | $-CH_3$ | Cl |
| 34 | $-CH_2-C_6H_5$ | $-(CH_2)_{17}-CH_3$ | $-CH_3$ | $-(CH_2)_3CH_3$ | Cl |
| 35 | $-CH_2-C_6H_5$ | $-(CH_2)_{17}-CH_3$ | $-(CH_2)_3CH_3$ | $-CH_3$ | Cl |
| 36 | $-CH_2-C_6H_5$ | $-(CH_2)_{17}-CH_3$ | $-CH_3$ | *iso*-Butyl | Cl |
| 37 | $-CH_2-C_6H_5$ | $-(CH_2)_{17}-CH_3$ | *iso*-Butyl | $-CH_3$ | Cl |
| 38 | $-CH_2-C_6H_5$ | $-(CH_2)_{17}-CH_3$ | *tert*-Butyl | $-CH_3$ | Cl |

(continued)

| Cpd n° | R1 | R2 | R3 | R4 | X |
|---|---|---|---|---|---|
| 39 | $-(CH_2)_2-C_6H_5$ | $-(CH_2)_{17}-CH_3$ | $-CH_3$ | $-CH_3$ | Cl |
| 40 | $-(CH_2)_2-C_6H_5$ | $-(CH_2)_{17}-CH_3$ | $-CH_3$ | $-CH_2CH_3$ | Cl |
| 41 | $-(CH_2)_2-C_6H_5$ | $-(CH_2)_{17}-CH_3$ | $-CH_2CH_3$ | $-CH_3$ | Cl |
| 42 | $-(CH_2)_2-C_6H_5$ | $-(CH_2)_{17}-CH_3$ | $-CH_3$ | $-(CH_2)_2CH_3$ | Cl |
| 43 | $-(CH_2)_2-C_6H_5$ | $-(CH_2)_{17}-CH_3$ | $-(CH_2)_2CH_3$ | $-CH_3$ | Cl |
| 44 | $-(CH_2)_2-C_6H_5$ | $-(CH_2)_{17}-CH_3$ | $-CH_3$ | $-CH(CH_3)_2$ | Cl |
| 45 | $-(CH_2)_2-C_6H_5$ | $-(CH_2)_{17}-CH_3$ | $-CH(CH_3)_2$ | $-CH_3$ | Cl |
| 46 | $-(CH_2)_2-C_6H_5$ | $-(CH_2)_{17}-CH_3$ | $-CH_3$ | $-(CH_2)_3CH_3$ | Cl |
| 47 | $-(CH_2)_2-C_6H_5$ | $-(CH_2)_{17}-CH_3$ | $-(CH_2)_3CH_3$ | $-CH_3$ | Cl |
| 48 | $-(CH_2)_2-C_6H_5$ | $-(CH_2)_{17}-CH_3$ | $-CH_3$ | *iso*-Butyl | Cl |
| 49 | $-(CH_2)_2-C_6H_5$ | $-(CH_2)_{17}-CH_3$ | *iso*-Butyl | $-CH_3$ | Cl |
| 50 | $-(CH_2)_2-C_6H_5$ | $-(CH_2)_{17}-CH_3$ | $-CH_3$ | *tert*-Butyl | Cl |
| 51 | $-(CH_2)_2-C_6H_5$ | $-(CH_2)_{17}-CH_3$ | *tert*-Butyl | $-CH_3$ | Cl |

The quaternary ammonium inhibits the Protein Kinase C activity *in vitro* and/or *in vivo*; preferably inhibits specifically Protein Kinase C alpha (PKC-$\alpha$) involved into inflammation and healing pathways. Methods for measuring the PKC activity are well-known from the skilled artisan. An example of such a method is disclosed in the Examples below. Briefly, said methods include differential expression analysis with PCR array, ELISA test, flow cytometry and the evaluation of cell viability.

According to one embodiment, the quaternary ammonium is selected from the group of quaternary ammonium halide wherein the nitrogen atom of the ammonium group is substituted by at least one alkyl group having at least 12 carbons atoms, preferably, comprising 14, 16 or 18 carbons atoms; and at least one substituted or unsubstituted alkylphenyl or benzyl group, preferably C1-C4 alkylphenyl group or benzyl group, more preferably benzyl group.

In a preferred embodiment, said inhibitor of PKC-$\alpha$ is only C16-alkyl quaternary ammonium halide; more preferably cetalkonium chloride (CKC), C16-alkyl cetrimide or C16-alkyl cetylpyridinium chloride.

The invention also relates to a composition, a pharmaceutical composition or a medicament comprising an inhibitor of PKC alpha of formula (I) as defined above.

According to one embodiment, the composition, the pharmaceutical composition or the medicament comprises a sterile aqueous phase selected from water, physiologic medium or buffer; more preferably the sterile aqueous phase is physiologic medium, tromethamine hydrochloride buffer or phosphate-buffered saline (PBS).

According to one embodiment, the composition, the pharmaceutical composition or the medicament comprises an oily phase selected from the group of mineral oils, monocaprylate sorbitan, squalene, propylene glycol esters, almond oil, babassu oil, blackcurrant seed oil, borage oil, canola oil, castor oil, coconut oil, cod liver oil, corn oil, cottonseed oil, evening primrose oil, fish oil, grapeseed oil, mustard seed oil, oat oil, olive oil, palm kernel oil, palm oil, peanut oil, rapeseed oil, safflower oil, sesame oil, walnut oil, wheat germ oil, hydrogenated castor oil, hydrogenated palm oil, hydrogenated cottonseed oil, hydrogenated palm oil, hydrogenated soybean oil, partially hydrogenated soybean oil, hydrogenated vegetable oil, isopropyl myristate, isopropyl isostearate, isopropyl palmitate, modified triglycerides, caprylic/capric glycerides, caprylic/capric triglyceride, fractionated triglycerides, glyceryl tricaprate, glyceryl tricaproate, glyceryl tricaprylate, glyceryl tricaprylate/caprate, glyceryl tricaprylate/caprate, glyceryl tricaprylate/caprate/laurate, glyceryl tricaprylate/caprate/linoleate, glyceryl tricaprylate/caprate/stearate, glyceryl trilaurate, glyceryl trilinoleate, glyceryl trilinolenate, glyceryl trioleate, glyceryl triundecanoate , linoleic glycerides, saturated polyglycolized glycerides, synthetic medium chain triglyceride containing primarily $C_8$-$C_{12}$ fatty acid chains, medium chain triglycerides (MCT), long chain triglycerides, modified triglycerides, fractionated triglycerides, silicones, phospholipids and mixtures thereof; long chain triglycerides as used herein includes a glycol triester where the acid moieties are saturated, monounsaturated or polyunsaturated fatty acids with a chain of 14 to 20 carbon atoms. Typical fatty acid moieties are oleic acid, stearic acid and linoleic acid. Suitably the hydrophobic phase comprises one or more monoglycerides, diglycerides, triglycerides or mixtures thereof. Preferably the one or more monoglycerides, diglycerides, triglycerides are glycerol esters of fatty acids containing from 4 to 22 carbon atoms.

Preferably, the oily phase is selected from short chain (C4 to C6) fatty acids mono-, di-and triesters of glycerol,

medium chain (C8 to C12) fatty acids mono-, di- and triesters of glycerol, long chain (C14 and more) saturated fatty acids mono-, di- and triesters of glycerol, long chain (C14 and more) unsaturated fatty acids mono-, di- and triesters of glycerol, vegetable oils, almond oil, babassu oil, blackcurrant seed oil, borage oil, canola oil, castor oil, coconut oil, cod liver oil, corn oil, cottonseed oil, evening primrose oil, fish oil, grapeseed oil, mustard seed oil, oat oil, olive oil, palm kernel oil, palm oil, peanut oil, rapeseed oil, safflower oil, sesame oil, shark liver oil, squalane, soybean oil, sunflower oil, walnut oil, wheat germ oil, hydrogenated castor oil, hydrogenated coconut oil, hydrogenated cottonseed oil, hydrogenated palm oil, hydrogenated soybean oil, partially hydrogenated soybean oil, hydrogenated vegetable oil, fatty acids esters (e.g. ethyl oleate, isopropyl myristate, isopropyl palmitate, isopropyl stearate, isopropyl isostearate...), short chain (C4 to C6) fatty acids mono-and diesters of propylene glycol, medium chain (C8 to C12) fatty acids mono- and diesters of propylene glycol, long chain (C14 and more) saturated fatty acids mono- and diesters of propylene glycol, long chain (C14 and more) unsaturated fatty acids mono-and diesters of propylene glycol, fatty alcohol (e.g. myristil alcohol, oleyl alcohol...), branched fatty alcohol (octyldodecanol...), silicone oils, mineral oils, petrolatum, vitamin E, vitamin E acetate, tocopherol, tocopherol acetate, saturated fatty acids, unsaturated fatty acids and phospholipids.

In an embodiment, the oily phase is free of phospholipids.

In an embodiment, the oily phase consists of MCT.

In an embodiment, the oily phase consists of a mixture of mineral oils, preferably a mixture of heavy and light mineral oils, more preferably 50% heavy mineral oil and 50% light mineral oil.

According to one embodiment, the composition, the pharmaceutical composition or the medicament is preferably stable at room temperature.

According to one embodiment, the composition, the pharmaceutical composition or the medicament is sterile. According to a preferred embodiment, the composition, the pharmaceutical composition or the medicament of the invention is sterilized by suitable and approved method (e.g. filtration, moist heat, dry heat, gamma or beta ray, ethylene oxide).

According to one embodiment, the composition, the pharmaceutical composition or the medicament of the invention further comprises excipients, diluents and/or carriers selected with regard to the intended route of administration. Examples of such excipients, diluents and/or carriers include, but are not limited to, the group of lubricating agents, wetting agents, emulsifying and suspending agents, dispersing agents, desintegrants, bulking agents, fillers, preserving agents, sweetening agents, flavoring agents, flow regulators, release agents; more preferably, the composition, the pharmaceutical composition or the medicament of the invention comprises quaternary ammonium of formula (I) as hereinabove described in association with an ophthalmic acceptable excipient.

According to one embodiment, the composition, the pharmaceutical composition or the medicament of the invention further comprises non-ionic surfactants selected from the group chosen among polyoxyethylene sorbitan fatty acid ester, castor oil derivatives, polyoxyethylene glycol hydrogenated castor oil, polyoxyethylene glycol castor oil, a sorbitan fatty acid ester (e.g. sorbitan monolaurate, sorbitan monooleate), a block copolymer of ethylene oxide and propylene oxide (e.g. poloxamer 188), poloxamers, tyloxapol, polysorbates and vitamin E polyethylene glycol succinate.

The present invention also relates to a process for manufacturing a composition, a pharmaceutical composition or a medicament comprising a quaternary ammonium used as an inhibitor of PKC-$\alpha$, comprising mixing:

- an inhibitor of PKC alpha of formula (I) selected from the group of quaternary ammonium halide wherein the nitrogen atom of the ammonium group is substituted by at least one alkyl group having at least 12 carbons atoms; preferably, cetalkonium chloride (CKC), C16-alkyl cetrimide or C16-alkyl cetylpyridinium chloride; more preferably cetalkonium chloride (CKC),
- with an oily phase selected from the group comprising mineral oils, monocaprylate sorbitan, squalene, propylene glycol esters, almond oil, babassu oil, blackcurrant seed oil, borage oil, canola oil, castor oil, coconut oil, cod liver oil, corn oil, cottonseed oil, evening primrose oil, fish oil, grapeseed oil, mustard seed oil, oat oil, olive oil, palm kernel oil, palm oil, peanut oil, rapeseed oil, safflower oil, sesame oil, walnut oil, wheat germ oil, hydrogenated castor oil, hydrogenated palm oil, hydrogenated cottonseed oil, hydrogenated palm oil, hydrogenated soybean oil, partially hydrogenated soybean oil, hydrogenated vegetable oil, isopropyl myristate, isopropyl isostearate, isopropyl palmitate, modified triglycerides, caprylic/capric glycerides, caprylic/capric triglyceride, fractionated triglycerides, glyceryl tricaprate, glyceryl tricaproate, glyceryl tricaprylate, glyceryl tricaprylate/caprate, glyceryl tricaprylate/caprate, glyceryl tricaprylate/caprate/laurate, glyceryl tricaprylate/caprate/linoleate, glyceryl tricaprylate/caprate/stearate, glyceryl trilaurate, glyceryl trilinoleate, glyceryl trilinolenate, glyceryl trioleate, glyceryl triundecanoate, linoleic glycerides, saturated polyglycolized glycerides, synthetic medium chain triglyceride containing primarily C8-C12 fatty acid chains, medium chain triglycerides (MCT), long chain triglycerides, modified triglycerides, fractionated triglycerides, silicones, phospholipids and mixtures thereof; long chain triglycerides as used herein includes a glycol triester where the acid moieties are saturated, monounsaturated or polyunsaturated fatty acids with a chain of 14 to 20 carbon atoms. Typical fatty acid moieties are oleic acid, stearic acid and linoleic acid. Suitably the hydrophobic phase comprises one or more monoglycerides, diglycerides, triglycerides or mixtures thereof. Preferably the one or more

monoglycerides, diglycerides, triglycerides are glycerol esters of fatty acids containing from 4 to 22 carbon atoms.

Preferably, the oily phase is selected from short chain (C4 to C6) fatty acids mono-, di-and triesters of glycerol, medium chain (C8 to C12) fatty acids mono-, di- and triesters of glycerol, long chain (C14 and more) saturated fatty acids mono-, di- and triesters of glycerol, long chain (C14 and more) unsaturated fatty acids mono-, di- and triesters of glycerol, vegetable oils, almond oil, babassu oil, blackcurrant seed oil, borage oil, canola oil, castor oil, coconut oil, cod liver oil, corn oil, cottonseed oil, evening primrose oil, fish oil, grapeseed oil, mustard seed oil, oat oil, olive oil, palm kernel oil, palm oil, peanut oil, rapeseed oil, safflower oil, sesame oil, shark liver oil, squalane, soybean oil, sunflower oil, walnut oil, wheat germ oil, hydrogenated castor oil, hydrogenated coconut oil, hydrogenated cottonseed oil, hydrogenated palm oil, hydrogenated soybean oil, partially hydrogenated soybean oil, hydrogenated vegetable oil, fatty acids esters (e.g. ethyl oleate, isopropyl myristate, isopropyl palmitate, isopropyl stearate, isopropyl isostearate...), short chain (C4 to C6) fatty acids mono-and diesters of propylene glycol, medium chain (C8 to C12) fatty acids mono- and diesters of propylene glycol, long chain (C14 and more) saturated fatty acids mono- and diesters of propylene glycol, long chain (C14 and more) unsaturated fatty acids mono-and diesters of propylene glycol, fatty alcohol (e.g. myristil alcohol, oleyl alcohol...), branched fatty alcohol (octyldodecanol...), silicone oils, mineral oils, petrolatum, vitamin E, vitamin E acetate, tocopherol, tocopherol acetate, saturated fatty acids, unsaturated fatty acids and phospholipids.

In an embodiment, the oily phase is free of phospholipids.

In an embodiment, the oily phase consists of MCT.

In an embodiment, the oily phase consists of a mixture of mineral oils, preferably a mixture of heavy and light mineral oils, more preferably 50% heavy mineral oil and 50% light mineral oil.

According to one embodiment, the process further comprises a step of mixing the oily phase comprising quaternary ammonium of formula (I), with an aqueous phase.

According to the present invention, the composition, the pharmaceutical composition or the medicament as hereinabove described, is useful for preventing or treating a PKC alpha related diseases in a patient; especially in the treatment of eye diseases or conditions including corneal and conjunctival lesions, more specifically wounds from various origins (surgery, infections, ocular diseases, ocular surface complications of systemic diseases, etc...). In the meaning of the present invention, eye diseases or eye conditions refers to a wide variety of ocular conditions namely glaucoma, ocular inflammatory conditions such as keratitis, uveitis, post-surgical inflammation, allergy, ocular infections, keratoconjuctivis sicca (i.e. Dry Eye Syndrome or Chronic Dry Eye Disease (CDED)).

In a preferred embodiment, said composition is useful for the treatment of keratoconjuctivis sicca (i.e. Dry Eye Syndrome or Chronic Dry Eye Disease (CDED), diabetic keratopathy or neurotrophic keratopathy.

In a preferred embodiment, the composition as hereinabove described, is useful for the treatment of glaucoma, anterior uveitis.

According to an embodiment of the present invention, the composition, the pharmaceutical composition or the medicament further contains active ingredient; preferably chosen among active substance which is selected from the group comprising:

**antiallergenics** such as sodium cromoglycate, antazoline, chlorpheniramine, cetirizine, olapatadine, ketotifen, azelastine, emedastine, levocabastine, terfenadine, loratadine;

**anti-inflammatories** such as cortisone, hydrocortisone, hydrocortisone acetate, dexamethasone, dexamethasone 21-phosphate, dexamethasone palmitate, fluorocinolone, prednisone, methylprednisone, prednisolone acetate, fluoromethalone, triamcinolone, betamethasone, loteprednol, flumethasone, beclomethasone, difluprednate and triamcinolone acetonide and their derivatives;

**non-steroidal anti-inflammatories** such as salicylate, indomethacin, ibuprofen, diclofenac, flurbiprofen, oxican, piroxicam and COX2 inhibitors such as rofecoxib ,nimesulide, nepafenac;

**beta adrenergic blockers** such as timolol and salts thereof maleate, levobunolol hydrochloride and betaxolol hydrochloride, betaxolol, atenolol, befundol, metipranolol, forskolin, carteolol;

**cytokines, interleukins ;**

**cyclosporines** such as cyclosporine A, B, C, D, E, F, G, H, I, J, K, L, M, N, O, P, Q, R, S, T, U, V, W, X, Y, Z and derivatives thereof;

**sirolimus, tacrolimus;**

**antioxidants** such as lutein, vitamins, especially vitamin A, coenzyme Q10, polyunsaturated fatty acids and derivatives thereof;

**inhibitors of carbonic anhydrase** such as brinzolamide, dorzolamide, acetazolamide, methazolamide, dichlorophenamide;

**antivirals,** such as idoxuridine, trifluorotymidine, acyclovir, valaciclovir, ganciclovir, cidofovir and interferon;

**antibiotics** such as aminoglycosides, carbacephem, carbapenems, cephalosporins, glycopeptides, penicillins, polypeptides, quinolones, sulfonamides, tetracyclines, chlortetracycline, bacitracin, neomycin, polymyxin, gramicidin, cephalexin, oxytetracycline, chloramphenicol, kanamycin, rifampicin, tobramycin, gentamycin, ciprofloxacin, aminosides, erythromycin, ceftazidime, vancomycine, imipeneme; macrolides, clarithromycine, azithromycine, fluroroquinolones;

**antifungals** such as polyene antibiotics, azole derivatives, imidazole, triazole, allylamines, amphotericin B, econazole, voriconazole and miconazole;

**antibacterials** such as sulfonamides, sulfadiazine, sulfacetamide, sulfamethizole and sulfisoxazole, nitrofurazone and sodium propionate;

and/or their derivatives; and/or their prodrugs; and/or their precursors; and/or acceptable salts thereof; alone or in combination.

The present invention also relates to a method for inhibiting a protein kinase C (PKC), wherein the method comprises administering an effective amount of quaternary ammonium of formula (I); preferably selected from the group of quaternary ammonium halide wherein the nitrogen atom of the ammonium group is substituted by at least one alkyl group having at least 12 carbons atoms, preferably 14, 16 or 18 carbons atoms; and at least one substituted or unsubstituted alkylphenyl or benzyl group, preferably C1-C4 alkylphenyl group or benzyl group, more preferably benzyl group; or of a composition, a pharmaceutical composition or a medicament of the invention. Preferably, said quaternary ammonium is cetalkonium chloride (CKC), C16-alkyl cetrimide or C16-alkyl cetylpyridinium chloride; more preferably cetalkonium chloride (CKC).

The present invention also relates to a method for treating a PKC related disease in a subject in need thereof, comprising administering to the subject an effective amount of a quaternary ammonium halide as hereinabove described.

According to a preferred embodiment, the present invention relates to a method of accelerating or promoting healing of damaged ocular tissue or an ocular wound in a subject.

In one embodiment of the invention, the subject is an animal; preferably a mammal; more preferably a human wherein the human is a male or a female.

According to an embodiment, the composition, the pharmaceutical composition or the medicament of the invention is in a form adapted to be administered on tissue and/or organ; preferably selected from the group comprising powder, ointments, gels, patch, film or any solids forms suitable for the preparation of a solid form to prior use, such as for instance, powder, liposomal forms and the like.

In one embodiment, the composition, the pharmaceutical composition or the medicament of the invention is topically administered in eye; preferably through any form selected from solution, dispersion, emulsion, eye drops, artificial tears, ointments, gel, patch, film or suspension suitable for ophthalmic use; more preferably by eye drops.

According to an embodiment, the composition, the pharmaceutical composition or the medicament of the invention is in a form adapted for injection; preferably selected from the group comprising solutions such as sterile aqueous solutions, dispersions, emulsions, suspensions, solids forms suitable for using to prepare solutions or suspensions upon the addition of a liquid to prior use, such as for instance, powder, liposomal forms and the like.

In a preferred embodiment, the concentration of said inhibitor of PKC-$\alpha$ in the composition, the pharmaceutical composition or the medicament of the present invention is ranging from greater than 0 % to 0.3%; preferably is 0.2%; more preferably is 0.002 or 0.005 %.

In one embodiment, the therapeutically effective amount of said inhibitor of PKC-$\alpha$ may be determined in consideration of some parameters such as the age, weight, sex, difference in diseases and severity of the condition of individual patients. It would be understood that the specific posology for any particular subject or patient may be varied and will depend upon some parameters as hereinabove described. It would be understood that the specific posology for any particular subject or patient may vary and may depend upon some parameters as hereinabove described. In one embodiment, the administration is once to eight times a day, with 1-10 drops at each administration.

According to an embodiment, the composition, the pharmaceutical composition or the medicament comprising a quaternary ammonium for use as an inhibitor of PKC-$\alpha$ is packaged in unitary doses.

In another embodiment, the composition, the pharmaceutical composition or the medicament is packaged in suitable multidose containers.

This invention also relates to a method for treating an eye condition, comprising applying an inhibitor of PKC alpha of formula (I) as described above, or a pharmaceutical composition as described above containing the same, onto the eye of a subject.

According to one embodiment, said method accelerates or promotes healing of damaged ocular tissue or an ocular wound in a subject.

According to one embodiment, the subject is an animal; preferably a mammal; more preferably a human wherein the human is a male or a female.

According to one embodiment, said method comprises a step of topical administration in eye: preferably through any form selected from solution, dispersion, emulsion, eye drops, artificial tears, ointments, gel, patch, film or suspension suitable for ophthalmic use; more preferably by eye drops.

In one preferred embodiment, said topical administration is in a form adapted to be administered on tissue and/or organ; preferably selected from the group comprising powder, ointments, gels, patch, film or any solids forms suitable for the preparation of a solid form to prior use, such as for instance, powder, liposomal forms and the like.

In one preferred embodiment, said topical administration is in a form adapted for injection; preferably selected from the group comprising solutions such as sterile aqueous solutions, dispersions, emulsions, suspensions, solids forms suitable for using to prepare solutions or suspensions upon the addition of a liquid to prior use, such as for instance, powder, liposomal forms and the like.

In one preferred embodiment, said method comprises the administration of the concentration of inhibitor of PKC-α ranging from greater than 0 % to 0.3% w/w, in weight to the total weight of the composition; preferably is 0.2%; more preferably is 0.002 or 0.005 %.

**DEFINITIONS**

In the present invention, the following terms have the following meanings:

**"Alkyl"** refers to compound of formula $C_nH_{2n+1}$, wherein n is a number greater than or equal to 1. Generally, alkyl groups of this invention comprise from 1 to 20 carbon atoms. Alkyl groups may be linear or branched and may be substituted. Examples of alkyl groups are methyl, ethyl, n-propyl, i-propyl, butyl and its isomers (e.g. n-butyl, i-butyl and t-butyl); pentyl and its isomers, hexyl and its isomers;

**"Alkene"** refers to any linear or branched hydrocarbon chain having at least one double bond;

**"Alkyne"** refers to any linear or branched hydrocarbon chain having at least one triple bond;

**"Aryl"** refers to any polyunsaturated, aromatic hydrocarbyl group having a single ring (i.e. phenyl) or multiple aromatic rings fused together (e.g. naphtyl) or linked covalently, typically containing 6 to 50 atoms; preferably 6 to 10, wherein at least one ring is aromatic;

**"Alkylaryl"** refers to any alkyl group joined to any aryl group;

**"Alkylbenzyl"** refers to any benzyl group linked to any alkyl group;

**"Alkylphenyl"** refers to any phenyl group linked to any alkyl group;

**"Alkoxy"** refers to any alkyl group singular bonded to oxygen;

**"Amido"** refers to the functional group N-C(=O);

**"Amino"** refers to a $-NH_2$ group or any group derived thereof by substitution of one nor two hydrogen atom by an organic aliphatic or aromatic group;

**"Benzyl"** refers to a functional group of formula $C_6H_5-CH_2-$;

**"Heteroalkyl"** refers to alkyl group having at least one atom that is not carbon or hydrogen; preferably, said atom is selected from N, S, P or O;

**"Heteroaryl"** refers to aryl group having at least one atom that is not carbon or hydrogen; preferably, said atom is selected from N, S, P or O;

**"BAK C12"** refers to benzododecinium chloride (CAS 139-07-1);

**"BAK C14"** refers to myristalkonium chloride (CAS 139-08-2);

**"Carboxy"** refers to the functional group C(=O)OH;

**"CKC"** refers to cetalkonium chloride (CAS 122-18-9);

**"C14-alkyl ammonium halides"** refers to means ammonium halides in which the nitrogen atom of the ammonium group is substituted by at least one alkyl group having at least 14 carbon atoms;

**"Cationic emulsions"** refers to emulsions having a positive zeta potential, preferably a zeta potential higher to 10 mV;

**"Cyano"** refers to a functional group consisting of a carbon atom triple-bonded to a nitrogen atom;

**"Ester"** refers to the functional group -C(=O)O-;

**"Healing agent"** refers to a compound which initiates, promotes, improves, enhances or contributes to the natural healing process of lesions in the human body;

**"Hydroxyl"** refers to the functional group -OH;

**"Inflammation"** refers to a part of the complex biological response of tissues to harmful stimuli, such as pathogens, damaged cells, or irritants. Controlled inflammation is a protective attempt by the organism to remove the injurious stimuli and to initiate the healing process;

**"Inhibitor"** refers to a natural or synthetic compound that has a biological effect to inhibit or significantly reduce or down-regulate the expression of a gene and/or a protein or that has a biological effect to inhibit or significantly reduce the biological activity of a protein;

**"Long alkyl chain"** refers to alkyl moieties having at least 12 carbon atoms;

**"MCT"** or **"Medium-chain triglycerides"** refers to compound containing 6-12 carbon fatty acid esters of glycerol;

**"Nitro"** refers to the functional group N(O)=O;

**"Phenyl"** refers to a functional group of formula $C_6H_5$-;

**"PKC"** or **"Protein Kinase C"** refers to a family of kinase enzymes known to regulate the majority of cellular pathways, especially those involved in signal transduction;

**"Ophthalmic"** as used herein in connection with a composition or emulsion, refers to a composition or emulsion intended to be administered to the eye of a subject and which presents a pharmaceutical effect;

**"Oxo"** refers to the functional group C=O;

**"Quaternary ammonium"** refers to ammonium compounds of general formula $N^+(R)_4$ wherein R is an alkyl or an aryl group and wherein the nitrogen atom is substituted by only one or at least one alkyl group having at least 12 carbon atoms ; quaternary ammonium compounds also, but not exclusively, include n-alkyl dimethyl benzyl ammonium chloride also called benzalkonium chloride (hereinafter also referred to as BAK or ADBAC) ; n-alkyl dimethyl benzyl ammonium bromide; n-alkyl trimethyl ammonium bromide (also referred to as ATAB), n- alkyl meaning an alkyl group of at least 12 carbon atoms;

**"Treating"** or **"treatment"** or **"alleviation"** refers to both therapeutic treatment and prophylactic or preventative measures; wherein the object is to prevent or slow down (lessen) the targeted pathologic condition or disorder.

Those in need of treatment include those already with the disorder as well as those prone to have the disorder or those in whom the disorder is to be prevented. A subject or mammal is successfully "treated" for an infection if, after receiving a therapeutic amount of an antibody according to the methods of the present invention, the patient shows observable and/or measurable reduction in or absence of one or more of the following: reduction in the number of pathogenic cells; reduction in the percent of total cells that are pathogenic; and/or relief to some extent, one or more of the symptoms associated with the specific disease or condition; reduced morbidity and mortality, and improvement in quality of life issues. The above parameters for assessing successful treatment and improvement in the disease are readily measurable by routine procedures familiar to a physician.

**ABBREVIATIONS**

**BAK:** benzalkonium chloride

**CKC:** cetalkonium chloride

**DMSO:** dimethylsulfoxide

**LPS:** Lipopolysaccharide

**MTT:** 3-(4,5-Dimethylthiazol-2-yl)-2,5-Diphenyltetrazolium Bromide

**NHEK:** Normal Human Epidermal Keratinocytes

**NF-κB:** Nuclear Factor-kappa B

**PKC:** Protein Kinase C

**PMA:** phorbol 12-myristate 13-acetate

**RNA:** ribonucleic acid

**RT:** room temperature

**RT-qPCR:** Reverse Transcriptase-quantitative Polymerase Chain Reaction

**TNF:** Tumor Necrosis Factor

**MATERIALS AND METHODS**

1. **Biologic models**

<u>CD4+ T-helper cell</u>

- Cell type : CD4+ T-helper cell purified from mononucleated blood cell
- Culture conditions :

  - 37°C, 5% $CO_2$
  - RPMI 1640 complemented with

    L-glutamine 2 mmol/L
    Penicillin 50 U/ml-streptomycine 50 μg/ml
    Inactivated fetal bovin serum 10%

Peripheral blood mononucleated cell (PBMC)

- Cell type : PBMC isolated from peripheral total blood (Ficoll®) (52-aged female donor)
- Culture conditions :
    - 37°C, 5% $CO_2$
    - RPMI complemented with
    L-glutamine 2 mmol/L
    Penicillin 100 U/ml-streptomycine 100 µg/ml
    Inactivated fetal bovin serum 10% (v/v)

**2. Tested compounds and stimulants/inducers**

| Tested compound | Aspect/stocking | Tested concentrations | |
|---|---|---|---|
| | | Pre-incubation 30 min | Incubation |
| **Emulsion with 0.002% CKC** | ▪ Liquid<br>▪ Stocking at RT | 10% | 0%, 0.001% and 0.01% |
| **Emulsion with 0.005% CKC** | ▪ Liquid<br>▪ Stocking at RT | 10% | 0%, 0.001% and 0.01% |

| stimulants/inducers | Stocking solution | Tested concentration |
|---|---|---|
| Anti-CD3 + Anti-CD28 | 1 mg/ml | 10 µg/ml + 3 µg/ml |
| | 1 mg/ml | |

**3. Cell cultures and treatments**

| Biologic models | References | Stimulators /inducers | Pre-incubation | Incubation | Tests |
|---|---|---|---|---|---|
| CD4+ T-helper | Cyclosporine A | Anti-CD3 + Anti-CD28 | 30 min | 24h | PCR array TNF-α titration alarmBlue® assay |
| | | | 30 min | 72h | IL-17 titration alarmBlue® assay |
| | Cyclosporine A | Anti-CD3 + Anti-CD28 | 30 min | 24h | TNF-α titration alarmBlue® assay |

**3.1. CD4+ T- helper cells**

CD4+ T- helper cells were pre-incubated during 30 min in a culture medium containing or not (blank experiment) tested compounds. Then, cells were activated by adducing anti-CD3 and anti-CD28 antibodies (respectively at 10 µg/ml and 3 µg/ml) and were incubated in culture medium containing compounds to test or the reference (here cyclosporine A at $10^{-6}$ M) during 24 hours (markers expression, delivered TNF-α) or 72 hours (delivered IL-17). Simultaneously, a control without activation was led. All experimental conditions were realized in n=3.

After incubation, culture surpernates were collected and stocked in order to titrate the quantity of delivered

TNF-α and IL-17 by ELISA titration and a viability test was led on cell layers according to a classical method of alarmBlue® reduction.

For the differential expression analysis, culture surpernates were eliminated and the plates were immediately freeze-dried at -80°C.

### 3.2. PBMC

PBMC cells were pre-incubated during 30 min in a culture medium containing or not (blank experiment) compounds to test. Then:

- Either the medium was replaced by a culture medium containing compounds to test or the reference (here dexamethasone at $10^{-7}$ M) in presence of the stimulant LPS at the concentration of 1 μg/ml and cells were incubated during 24 hours.
- Or cells were activated by adducing anti-CD3 and anti-CD28 antibodies (respectively at 10 μg/ml and 3 μg/ml) and were incubated in culture medium containing compounds to test or the reference (here cyclosporine A at $10^{-6}$ M) during 24 hours.

Simultaneously, a control without stimulant was led. All experimental conditions were realized in n=3.

After incubation, culture surpernates were collected and stocked at -80°C in order to titrate the quantity of delivered TNF-α and IL-8 by flow cytometry titration and a viability test was led on cell according to a classical method of alarmBlue® reduction.

For the differential expression analysis, culture surpernates were eliminated and the plates were immediately freeze-dried at -80°C.

## 4. Differential expression analysis - PCR array

Markers expression was evaluated by RT-qPCR on the RNA messengers extracted from cells of each treatment and from each tested cellular type (replicated cells were pooled before RNA extraction).

The analysis of genes expression was realized in n=2 thanks to the following PCR arrays:

- mQPA 16-CD4 + PBMC (GT131017)

These PCR arrays contain genes selected for their importance in inflammation pathways.

### 4.1. Inverse transcriptase

Total NRA of each sample was extracted with TriPure Isolation Reagent® according to a method given by the supplier. The quality and the quantity of NRA were evaluated by capillary electrophoresis (Bioanalyser 2100, Agilent). Potential contaminant DNA traces were eliminated by treatment with DNA-free system (Ambion). Complementary DNA (cDNA) were synthesized by inverse transcriptase of total RNA in presence of oligo(dT) and the Transcriptor Reverse Transcriptase enzyme (Roche). The obtained complementary DNA was quantified by spectrophotometry (Nanovue ; GE Healthcare); then, cDNA quantities were adjusted.

### 4.2. Quantitative PCR

Polymerase chain reaction (PCR) was led by quantitative PCR (Light Cycler; Roche Molecular Systems Inc.) and according to procedures recommended by the supplier.

The essay medium (10 μl) for each sample contained:

- 2, 5 μl of cDNA,
- Primers of the different used markers,
- Medium with enzyme (DNA polymerase), SYBR Green I marker and $MgCl_2$

### 4.3. Treatment of quantitative PCR data

Raw data were treated with Microsoft Excel® software.

Fluorescence incorporation in amplified DNA was continuously measured during PCR cycles. These measurements allow obtaining fluorescence intensity curves depending on PCR cycles and thus, evaluating the relative expression

for each marker.

Cycles number is determined from the output ports of fluorescence curves. For a same analyzed marker, later one sample exits (high cycles number), lower the initial number of RNA copies is.

The value of relative expression is calculated according to the following formula:

$$(1/2^{\text{number of cycles}}) \times 10^6$$

PCR array contains a reference gene ("housekeeping") used for data normalization.RPS28 gene (Ribosomal protein S28) or GAPDH (glyceraldehyde-3-phosphate dehydrogenase) was expressed in a constitutive manner and its expression level is not (or few) impacted by these treatment. Thus, the expression level of each marker is compared to those of reference gene.

### 5. Cell viability evaluation

After incubation, cell viability was evaluated by either MTT reduction test or alarmBlue® test.

#### 5.1. MTT reduction test

Cells were incubated en presence of MTT (tetrazolium salts) of which the transformation of blue crystals of formazan is proportional to succinate dehydrogenase activity (mitochondrial enzyme). After cells dissociation and formazan solubilization by adducing DMSO, optical density (OD) featuring live cells number and their metabolic activity, was measured by microplates reader at 540 nm (VERSAmax, Molecular Devices).

#### 5.2. alarmBlue® test

Cells were incubated with alarmBlue® (resazurine) reduced into resofurine by mitochondrial activity of active metabolic cells. This transformation is proportional to live cells number. The optical density (OD) featuring live cells number and their metabolic activity, was measured by microplates reader at 570 nm (VERSAmax, Molecular Devices).

### 6. Treatment of data

Raw data were treated with Microsoft Excel® software.

Intergroup comparisons were led with T test of unpaired bilateral Student. The statistical analyses can be studied if n≥5. However, calculated data are provided only for information purposes.

$$\%\text{ of inhibition} = \frac{Simulated\ average\ indicator - Value}{Simulated\ average\ indicator - Initial\ average\ indicator} \times 100$$

$$\text{Standard error of the average} = \frac{Standard\ deviation(Sd)}{\sqrt{n}}$$

$$\%\text{ of viability} = \frac{optical\ density\ of\ the\ compound}{optical\ density\ of\ the\ indicator} \times 100$$

## EXAMPLES

The present invention is further illustrated by the following examples.

Example 1: Inhibition of PKC by cetalkonium chloride (CKC)

The aim of this study is to evaluate the ability of CKC to specifically inhibit the protein kinase C alpha.

CKC was tested on a cellular assay to evaluate the protein kinase C inhibition (PKC). Four subtypes of PKC are evaluated ($\alpha$, $\beta$1, $\beta$2 and $\delta$). The results are shown Table 1.

*Table 1.* ***Cellular assay to evaluate the action of CKC on four subtypes of PKC.***

| Compound | IC 50 ($\mu$M) | | | |
|---|---|---|---|---|
| | **PKC alpha** | **PKC beta 1** | **PKC beta 2** | **PKC delta** |
| CKC | 4,29 | * | * | * |
| **IC 50 too high to be measured by apparatus.* | | | | |

The smaller IC50, the better the molecule will inhibit PKC. In view of the results, it appears that CKC specifically inhibits the protein kinase PKC$\alpha$ which is mainly involved into inflammatory and healing pathways.
Unexpectedly, the results evidence that CKC at low concentration inhibits on the PKC$\alpha$ and may act on inflammatory and healing pathways.

Example 2: *In vitro* inhibition of PKC by compositions containing CKC

The anti-inflammatory properties of compositions containing cetalkonium chloride have been studied by the Applicant. The anti-inflammatory properties were evaluated on the basis of the compositions effects on gene profile and on delivered markers involved in the immune response and the inflammation of biologic models.
The methods are as described above.

*Dose effect with emulsions comprising CKC*

Table 2 features the results obtained with two emulsions comprising 0.002% and 0.005% CKC.

*Table 2.* ***Cellular assay to evaluate the cell type stimulation from the action of emulsions comprising 0.002% or 0.005% CKC without association with a PKC inhibitor.***

| | | % of secretion inhibition | | |
|---|---|---|---|---|
| **Cell type stimulation** | **Biomarker** | **Emulsion with 0.002% CKC** | **Emulsion with 0.005% CKC** | **Positive control*** |
| T cells CD4+ anti-CD3/28 | IL17 | 65 | 98 | 100 |
| PBMC polynuclear/lymph B Anti-CD3/28 | TNF $\alpha$ | 49 | 62 | 90 |
| **Positive control compound is cyclosporine.* | | | | |

Unexpectedly, the results evidence first, that an emulsion with low concentration of CKC (0.002%) without any addition of further PKC inhibitor compound, inhibits the cell stimulation of the biomarkers T cells CD4+ anti-CD3/28 and PBMC polynuclear/lymph B Anti-CD3/28 involved in the inflammatory pathways.
Secondly, the Applicant has discovered that a concentration of 0.005% CKC in the emulsion allows achieving the best inhibition.
Thus, these results prove that CKC can be used as an efficient PKC inhibitor and consequently, as an efficient anti-inflammatory and wound-healing agent.

## Claims

1. Quaternary ammonium compound for use as an inhibitor of protein kinase C alpha, of formula I:

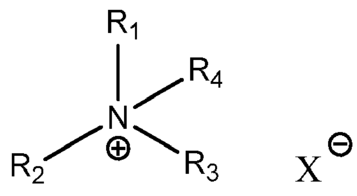

(I)

wherein:

$R_1$ is selected from the group of alkyl, alkene, aryl or alkylaryl group; preferably $R_1$ is a benzyl, alkylbenzyl or alkylphenyl group; more preferably benzyl or methylphenyl group; optionally substituted by hydroxyl, oxo, nitro, amido, carboxy, amino, cyano, alkoxy, alkyl, alkene, alkyne, ester or heteroalkyl group.
$R_2$ is an alkyl chain comprising at least 12 carbon atoms; preferably comprising 14, 16 or 18 carbon atoms;
$R_3$ or $R_4$ are identical or different and each is selected from an alkyl chain; preferably a C1-C4 alkyl chain; more preferably is methyl group.
X- is selected from an anion of a pharmaceutically acceptable acid or from halo group; preferably X- is selected from halo group; more preferably is $Cl^-$ or $Br^-$.

**2.** Quaternary ammonium compound according to claim 1, for use in preventing or treating a PKC alpha related disease in a patient in need thereof, where a therapeutically effective amount of compound of formula (I) is administered to the patient.

**3.** Quaternary ammonium compound for use according to claim 2, where the PKC alpha related disease is a wound healing condition.

**4.** Quaternary ammonium compound for use according to claim **2,** where the PKC alpha related disease is an inflammatory condition.

**5.** Quaternary ammonium compound for use according to anyone of claims **1** to **4,** selected from the group of quaternary ammonium halides.

**6.** Quaternary ammonium compound for use according to anyone of claims **1** to **5,** which is cetalkonium chloride.

**7.** Quaternary ammonium compound for use according to anyone of claims **2** to **6,** wherein the therapeutically effective amount is 0.002 % to 0.3% w/w, in weight to the total weight of the composition.

**8.** A composition comprising at least one quaternary ammonium compound for use as an inhibitor of protein kinase C alpha as described in anyone of claims **1** to **7,** in association with an pharmaceutically, preferably ophthalmically, acceptable excipient.

**9.** A composition according to claim **8,** wherein said composition is for use in the treatment of eye conditions.

**10.** A composition according to claim **8** or claim **9,** wherein said composition is for use in the treatment of ocular surface diseases, including but not limited to corneal and conjunctival lesions.

**11.** A composition according to anyone of claims **8** to **10,** which is topically administered through any form selected from solution, dispersion, emulsion, eye drops, artificial tears, gel, ointment, patch, film or suspension suitable for ophthalmic use.

**12.** A composition according to anyone of claims **8** to **11,** which is packaged in unitary doses or in multidose containers.

**13.** A medicament comprising a quaternary ammonium compound for use as an inhibitor of protein kinase C alpha as described in anyone of claims **1** to **7.**

Europäisches Patentamt
European Patent Office
Office européen des brevets

# EUROPEAN SEARCH REPORT

Application Number
EP 14 19 0172

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X | US 2008/026991 A1 (RABINOVICH-GUILATTI LAURA [IL] ET AL) 31 January 2008 (2008-01-31) * oil in water emulsions comprising quaternary ammonium compounds, the preferred being Benzalkonium Chloride and cetalkonium chloride, for use in the treatment of inflammatory ophthalmic diseases such as dry eye ("conjunctivitis sicca", i.e. dry inflammation of the conjunctiva). Concentrations as law as 0.00005% w/w are preferred. See paragraphs 1-3, 16-21, 29-35, 45, examples 1, 2; claims, in particular claims 1, 4, 21, 23. * ----- | 1,2,4-13 | INV. A61K31/14 A61P29/00 A61P27/00 A61P27/02 |
| X | US 2008/025941 A1 (RABINOVICH-GUILATT LAURA [FR] ET AL) 31 January 2008 (2008-01-31) * Oil in water emulsions comprising quaternary ammonium compounds, the preferred being benzalkonium chloride and cetalkonium chloride, for use in the treatment of inflammatory ophthalmic diseases such as keratitis, dry eye ("conjunctivitis sicca", i.e. dry inflammation of the conjunctiva), glaucoma etc. Concentrations as law as 0.0005% w/w. See paragraphs 1, 2, 11, 43, 44, 50, 50-53, examples; tables and claims. * ----- | 1,2,4-13 | TECHNICAL FIELDS SEARCHED (IPC): A61K |
| | -/-- | | |

The present search report has been drawn up for all claims

3

| Place of search | Date of completion of the search | Examiner |
|---|---|---|
| Munich | 6 February 2015 | Veronese, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 19 0172

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X | US 2009/018057 A1 (LAMBERT GREGORY [FR] ET AL) 15 January 2009 (2009-01-15)<br>* Ophthalmic compositions comprising benzalkonium chloride and an NSAID, for use in the treatment of ocular disorders associated with inflammation: see par. 1, 3, 5, 16, 33, 43; examples and claims 1, 19-21. *<br>----- | 1-6,8-13 | |
| X | JOURNAL OF PHARMACEUTICAL INVESTIGATION, vol. 42, no. 6, December 2012 (2012-12), pages 327-333, XP002734886, DOI: 10.1007/S40005-012-0043-2<br>* Benzalkonium chloride, loaded into a wound dressing, effectively promotes wound healing and decreases inflammatory cells in the wounds of animals: see abstract, section "results"; figures and conclusions on page 333 *<br>----- | 1-5,8, 10-13 | |
| X | DATABASE EMBASE [Online]<br>ELSEVIER SCIENCE PUBLISHERS, AMSTERDAM, NL;<br>1966,<br>OKADA S: "Management of open fractures; experimental study of surgical detergents",<br>XP002734887,<br>Database accession no. EMB-0007993644<br>* Benzalkonium chloride as an effective agent for promoting the healing of infected wounds: see abstract. *<br>-/-- | 1-4,8, 11-13 | TECHNICAL FIELDS SEARCHED (IPC) |

The present search report has been drawn up for all claims

3

| Place of search | Date of completion of the search | Examiner |
|---|---|---|
| Munich | 6 February 2015 | Veronese, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 19 0172

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| | & OKADA S: "Management of open fractures; experimental study of surgical detergents", JOURNAL OF THE JAPANESE ORTHOPAEDIC ASSOCIATION 1966, vol. 40, no. 3, 1966, pages 313-327, ISSN: 0021-5325 ----- | | |
| X | WO 2010/122490 A2 (VANANGAMUDI SULUR SUBRAMANIAM [IN]; SRINIVASAN MADHAVAN [IN]; CHULLIEL) 28 October 2010 (2010-10-28) * Compositions comprising benzalkonium chloride and cetrimide are effective for treating wound healing: see pages 16-24, table 10 and claims. * ----- | 1-4,8, 12,13 | |
| Y,D | "Anti-inflammatory activity of cationic lipids", BRITISH JOURNAL OF PHARMACOLOGY, vol. 122, 1997, pages 551-557, XP002111007, * Protein kinases C is involved in the anti-inflammatory action of quaternary ammonium compounds * ----- | 1-13 | TECHNICAL FIELDS SEARCHED (IPC) |
| Y | MARASCO C J ET AL: "SYNTHESIS AND BIOLOGICAL ACTIVITY OF NOVEL QUATERNARY AMMONIUM DERIVATIVES OF ALKYLGLYCEROLS AS POTENET INHIBITORS OF PROTEIN KINASE C", JOURNAL OF MEDICINAL CHEMISTRY, AMERICAN CHEMICAL SOCIETY, US, vol. 33, 1 January 1990 (1990-01-01), pages 985-992, XP001074074, ISSN: 0022-2623, DOI: 10.1021/JM00165A016 * Quaternary ammonium derivatives are potent inhibitors of Protein Kinase C and have anti-inflammatory action * ----- | 1-13 | |
| | -/-- | | |

The present search report has been drawn up for all claims

3

| Place of search | Date of completion of the search | Examiner |
|---|---|---|
| Munich | 6 February 2015 | Veronese, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 19 0172

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CATAISSON C ET AL: "Activation of cutaneous protein kinase C[alpha] induces keratinocyte apoptosis and intraepidermal inflammation by independent signaling pathways", THE JOURNAL OF IMMUNOLOGY, THE AMERICAN ASSOCIATION OF IMMUNOLOGISTS, US, vol. 171, no. 5, 1 September 2003 (2003-09-01), pages 2703-2713, XP002520096, ISSN: 0022-1767 * Activation of Protein kinase C alpha induces inflammation * ----- | 1-13 | |

TECHNICAL FIELDS SEARCHED (IPC)

The present search report has been drawn up for all claims

3

| Place of search | Date of completion of the search | Examiner |
|---|---|---|
| Munich | 6 February 2015 | Veronese, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 19 0172

This annex lists the patent family members relating to the patent documents cited in the above-mentioned European search report. The members are as contained in the European Patent Office EDP file on
The European Patent Office is in no way liable for these particulars which are merely given for the purpose of information.

06-02-2015

| Patent document cited in search report | Publication date | Patent family member(s) | Publication date |
|---|---|---|---|
| US 2008026991 A1 | 31-01-2008 | AU 2007278141 A1 | 31-01-2008 |
| | | CA 2659322 A1 | 31-01-2008 |
| | | EP 2049079 A2 | 22-04-2009 |
| | | EP 2404593 A1 | 11-01-2012 |
| | | JP 5441058 B2 | 12-03-2014 |
| | | JP 2009544667 A | 17-12-2009 |
| | | KR 20090047496 A | 12-05-2009 |
| | | US 2008026991 A1 | 31-01-2008 |
| | | US 2011201689 A1 | 18-08-2011 |
| | | WO 2008035246 A2 | 27-03-2008 |
| US 2008025941 A1 | 31-01-2008 | US 2008025941 A1 | 31-01-2008 |
| | | US 2008026013 A1 | 31-01-2008 |
| US 2009018057 A1 | 15-01-2009 | NONE | |
| WO 2010122490 A2 | 28-10-2010 | US 2012142631 A1 | 07-06-2012 |
| | | WO 2010122490 A2 | 28-10-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1620063 A
- US 20100278906 A
- WO 2012063237 A
- EP 2526953 A


### Non-patent literature cited in the description


- **BAUDOUIN, C. et al.** Preservatives in eye drops: the good, the bad and the ugly. *Prog. Retin. Eye Res.,* July 2010, vol. 29 (4), 312-334
- **DEBBASCH, C. et al.** Quaternary ammoniums and other preservatives contributions in oxidative stress and apoptosis on Chang conjunctival cells. *Inv. Ophtalmo. Vis. Sci.,* March 2001, vol. 42 (3), 642-652
- **VLACHY, N. et al.** Detremining the cytotoxicity of cationic surfactant mixtures on HeLa cells. *Colloids Surf. B Biointerfaces,* May 2009, vol. 70 (2), 278-280
- **FILION et al.** Anti-inflammatory activity of cationic lipids. *British Journal of Pharmacology,* 1997, vol. 122, 551-557
- **DAULL et al.** *Cornea,* 2013, vol. 32, 345-354
- **LIANG et al.** *Molecular Vision,* 2008, vol. 14, 204-214